# EUROPEAN PATENT SPECIFICATION

(11) **EP 4 501 387 B1**
(45) Date of publication and mention of the grant of the patent: **25.02.2026**
(21) Application number: 23188675.5
(22) Date of filing: 31.07.2023
(51) Int. Cl.: A61M 25/10, A61B 5/00, A61M 25/00, A61B 17/22, A61B 17/00

(54) **A DUAL CATHETER ARRANGEMENT AND SYSTEM FOR REPERFUSION OF AN ISCHEMIC TISSUE REGION VIA A CORONARY VESSEL**
DOPPELKATHETERANORDNUNG UND SYSTEM ZUR REPERFUSION EINER ISCHÄMISCHEN GEWEBEREGION ÜBER EIN KORONARGEFÄSS
AGENCEMENT DE CATHÉTER DOUBLE ET SYSTÈME DE REPERFUSION D'UNE RÉGION TISSULAIRE ISCHÉMIQUE PAR L'INTERMÉDIAIRE D'UN VAISSEAU CORONAIRE

(43) Date of publication of application: 05.02.2025
(73) Proprietor: Albert-Ludwigs-Universität Freiburg, 79098 Freiburg (DE); Osypka AG, 79618 Rheinfelden (DE)
(72) Inventor: Zgierski-Johnston, Callum, 79098 Freiburg (DE); Kohl, Peter, 79098 Freiburg (DE); Chleilat, Enaam, 79098 Freiburg (DE); Heiner, Wilfred Peter, 79618 Rheinfelden (DE)
(74) Representative: Rösler, Uwe

(56) References cited:
- EP-A2- 0 249 338
- WO-A1-2020/069216
- WO-A1-94/07446
- WO-A2-2008/042347
- WO-A2-99/37202
- US-A- 4 587 975
- US-A1- 2013 338 637
- US-A1- 2021 402 158
- US-B1- 6 398 773

## Description

### BACKGROUND OF THE INVENTION

### Field of the Invention

The invention relates to a dual catheter arrangement and a system in which the dual catheter arrangement serves as a central medical intervention tool for reperfusion of an ischemic tissue region via a coronary vessel.

### Description of the Prior Art

Myocardial ischemia occurs when blood flow to the heart muscle (myocardium) is reduced below levels required to serve tissue demand. This may be by a partial or complete blockage of a coronary artery by buildup of atherosclerotic plaques. If the atherosclerotic plaque ruptures, thrombi can form and can completely block the vessel, resulting in medical emergencies such as heart attack (myocardial infarction) or stroke.

Following myocardial ischemia caused by full or partial occlusion of a coronary artery, current treatment typically involves interventions that focus on 'reperfusion' of the ischemic tissue as quickly as possible, often followed by implantation of a stent into the vessel to prevent re-occlusion.

In this context, a catheter system is known from document US 2018/0280172 A1 which enables reperfusion of a coronary tissue region and delivering a stent for remaining in place. The catheter system comprising a proximal manifold, including an infusion port and a stent balloon inflation port, and a catheter, extending distally from the manifold and defining an infusion lumen in fluid communication with the infusion port, and an inflation lumen in fluid communication with the stent balloon inflation port. The catheter further provides a therapeutic agent port in fluid communication with a therapeutic agent lumen leading to the distal end of the catheter. Near the distal end of the catheter a stent balloon is arranged which is in fluid communication with the inflation lumen and surrounding the catheter. At the outer surface of the stent balloon a stent is attached for intravascular permanent placement.

Document US 10,952,883 B2 discloses the use of the catheter system previously described and a treatment for microvascular obstructions while avoiding reperfusion injuries. Initially the catheter including the stent will be navigated to a target location within a vessel using a guidewire first and advancing the catheter over the guidewire. Subsequently the stent balloon disposed within the stent will be inflated, thereby expanding the stent against a vessel wall at the target location and transiently occluding the vessel. Subsequently infusing an infusate into the vessel downstream of the balloon through the infusion lumen and analysing pressure and temperature for monitoring an effect of the infusion of the infusate. Finally the balloon will be deflated once a desired effect of the infusion of the infusate has been reached based on the analysis, while leaving the expanded stent in place as a safeguard against re-occlusion of the vessel.

Unfortunately, reperfusion after an ischemic period can itself lead to potentially fatal arrhythmias. These arrhythmias, induced by reperfusion, are mostly considered as a necessary evil that can be treated by way of controlled defibrillation, although defibrillation can cause additional tissue damage despite its efficacy.

Some of the more advanced approaches have explored perfusion with various washing solutions before restoring blood flow to reduce ischemia-related tissue damage and/or the likelihood of arrhythmias. However, the success of these attempts has remained limited in terms of reducing arrhythmogenesis.

Document US 2022/000498 A1 discloses a catheter system for removal of a thrombus from a vessel, comprising an inner catheter having a proximal end, an infusion segment having infusion fenestration, and a distal end, at which a distal encapsulation balloon is arranged through which passes a guide wire lumen. Further an outer sheath surrounds at least a portion of the inner catheter, having a distal end, at which a proximal encapsulation balloon is arranged, which is separated from the distal encapsulation balloon by a distance along the infusion segment. After intracorporeal insertion, both inflated balloons nestle fluid-tightly against the inner wall of a vessel to create a demarcated area between the two balloons, which is the exclusive area of interest, for purposes of local thrombus removal.

Document US 2021/0402158 A1 discloses an intravascular treatment system which is based on the same concept as the catheter system described above, i.e. having two inflatable balloons which are arranged along two catheter elements which can be guided into each other and which balloons, when inflated, separate a vessel area fluid-tight along a common catheter axis, within which a thrombus can be removed with subsequent stent application. Like the distal balloon in the previously described arrangement, the distally placed balloon has only a guidewire lumen passing through the balloon, but the distal balloon interfaces with the distal side in a fluid-tight manner when inflated.

Document EP 0 249 338 A2 discloses a retroperfusion catheter including a catheter body and a retroperfusion balloon at its distal end. The catheter also includes means to monitor the pressure at a location distally of the balloon as well as to infuse liquids and medicants downstream of the balloon rapidly and independently of the retroperfusion procedure. Also, the catheter body has electrode means proximally of the balloon to sense ECG activity directly within the blood vessel as well as to provide a means by which emergency pacing may be stimulated. The catheter construction includes an inner core formed from a soft flexible tube, which is surrounded by an overbraid which lends rigidity and torquability for the catheter, the overbraid being surrounded, in turn, by an outer jacket. The distal region of the catheter is bent to define a curve, which facilitates steering of the catheter as it is advanced through the patient's vascular system.

Document US 2017/0189654 A1 discloses a system for treating myocardial microvascular obstruction by introducing injectate into blood vessels affected by MVO at precise flow rates, while blocking retrograde flow, such that the natural pumping of the heart aids in forcing the injectate into the affected microvessels. Monitoring pressure distal of an occlusion balloon is used to determine treatment effectiveness and heart health. The system comprises a fluid pump assembly, a catheter having a distal end and a proximal end connected to said fluid pump. An inflatable balloon is arranged near said distal end, at which one pressure and temperature sensor is located to measure at least one of a pressure and/or temperature value inside the distal end of the catheter and a pressure and a temperature value outside the catheter distal to the balloon. On basis of such data acquired infusion parameters are generated to control activity of the fluid pump assembly.

Document US 2013/0338637 A1 discloses a catheter system and methods useful for cell therapy including a first catheter having a first lumen for accepting a treatment device, a second lumen for inflating a balloon, a pressure sensor for monitoring fluid pressure within the first lumen, and a flow restrictor such as a hemostasis valve for limiting the exchange of fluids into and out of the first lumen while treatment devices are present or exchanged in the first lumen.

Document US 6 398 773 B1 discloses aspiration catheters and methods for the treatment of an occlusion in a blood vessel. The catheters are provided with varying flexibility along the length of the shaft, such that they are soft and flexible enough to be navigated through the vasculature of a patient without causing damage, but are stiff enough to sustain the axial push required to position the catheter properly and to sustain the aspiration pressures.

Document WO 99/37202 A2 discloses a method and device for occluding a patient's ascending aorta, maintaining circulation of oxygenated blood in the patient and delivering cardioplegic fluid to arrest the patient's heart. An aortic occlusion catheter has an occluding member for occluding the ascending aorta. The aortic occlusion catheter passes through a cannula. Delivery of oxygenated blood is accomplished through either the cannula or the aortic occlusion catheter. Document EP 249 338 A2 discloses a retroperfusion catheter including a catheter body having a balloon mounted at its distal end. The balloon is shaped to expand into sealed, temporarily obstructing relation with the vein when inflated and to collapse to minimize obstruction to the cross-sectional flow area within the vein when deflated. The body has a main lumen with a distal outlet port at the distal tip of the main body. The inner tubular core of the main lumen is wrapped with a wire overbraid which provides for stiffness and the assembly is coated with a urethane outer jacket. The wire overbraid terminates short of the balloon so that the most distal end of the catheter remains soft and flexible.

### SUMMARY OF THE INVENTION

It is an object of the invention to provide a catheter system that allows a reliable treatment of myocardial ischemia without the subsequent occurrence of reperfusion arrhythmias. It is a further aspect of the invention to provide a catheter system that allows an easy and reliable catheter handling and to ensure that the catheter treated myocardial tissue region is set in a state that allows a sufficient grade of blood supply to the myocardium.

The invention is set out in the appended set of claims. Claim 1 addresses a dual catheter arrangement. Furthermore, a system for reperfusion of an ischemic tissue region via a coronary vessel comprising the dual catheter arrangement is defined in claim 17. The features which improve upon the idea of the approach in an advantageous manner are the subject matter of the dependent claims as well as the additional description with reference to the embodiments.

The invention is based on the finding from animal experiments and computer simulations performed by the inventors that a two-zone reperfusion method, in which a distal region of the ischemic tissue area is re-perfused with a normal physiological infusate earlier than a proximal tissue area, can significantly reduce arrhythmogenesis. This finding is based on the understanding of reasons for the formation of ischemia-reperfusion-related arrhythmias obtained in the animal studies as follows.

In the normal state, the electrical signals generated by the heart's primary pulse generator (sinus node) are transmitted in an orderly sequence to coordinate the heart's pumping activity. If there is a blockage in a coronary artery, insufficient oxygen is supplied to the muscle cells and acidification and an increase in extracellular potassium occurs. This results in an ischemic zone in the region of the heart muscle supplied by this artery. The consequences of this are reduced electrical excitability and a slowing down or complete blockade of electrical excitation in this region of the heart muscle.

If the vascular blockage is removed using conventional reperfusion, i.e. by means of a reperfusion catheter known per se, as described before, the stenosed cardiac vascular region is permanently dilated by placement of a stent, and the ischemic myocardial region is washed with either blood or a reperfusion solution, there is subsequently increased heterogeneity of electrophysiological properties, particularly in a border zone peripherally surrounding the reperfused myocardial region, which may act as a trigger for arrhythmias before subsequent full or partial recovery of the ischemic tissue.

In addition to this mechanism it turns out, that myocardium along the reperfused coronary artery returns to electrical excitability temporally before the bulk of the ischemic region, resulting in the formation of an electrical conduction pathway through the midst of the ischemic region treated by reperfusion. This phenomenon may be named PeriVascular Excitation Tunneling, abbreviated PVET, and may lead to reentrant arrhythmias.

To effectively counteract the aforementioned PVET mechanism for the occurrence of cardiac arrhythmias, the already briefly mentioned two-zone reperfusion technique is proposed which is abbreviated as SHIELD-EP, which means "Step-wise Haemodynamic recovery of IschEmic myocardium to Lessen Deleterious effects on cardiac ElectroPhysiology". Especially for performing SHIELD-EP an inventively designed dual- catheter arrangement is proposed as follows:
A dual catheter arrangement for reperfusion of an ischemic tissue region via a coronary vessel, comprising a primary balloon catheter having a lumen for inflation and deflation of a primary balloon disposed at a distal region of the primary balloon catheter and at least two separate lumens both extending throughout the primary balloon catheter and open at a distal end of the primary balloon catheter, one of both separate lumens having a connecting flange proximally for detachable, fluid-tight connection to a flushing source unit, and a secondary balloon catheter having a lumen for inflation and deflation of a secondary balloon disposed at a distal region of the secondary balloon catheter and an irrigation lumen having a connecting flange proximally for detachable, fluid-tight connection to a flushing source unit and an opening at a distal end of the secondary balloon catheter, which provides at least in an axial region along which the secondary balloon is disposed a means of radial reinforcement and which is arranged within the other one of the both lumens of the primary balloon catheter so that the secondary balloon catheter continues completely through the lumen of the primary balloon catheter and extends distally beyond the primary balloon catheter.

In a preferred embodiment the secondary balloon catheter is slidable arranged within the primary balloon catheter so that by sliding positioning of the secondary balloon catheter within the primary balloon catheter a final distance between primary and secondary balloon in a working position can be individually adjusted with respect to the respective anatomical conditions.

As will be described further in detail with reference to the illustrated embodiments, to perform the SHIELD-EP method, the distal end of the primary catheter is positioned in close proximity to the location of the stenosis so that the stenosed myocardial tissue region can be dilated by means of the primary balloon. The secondary catheter protrudes through the primary catheter, with a secondary catheter region protruding beyond the first catheter to lie within the ischemic tissue region, i.e. past the stenosed or otherwise blocked proportion of the vessel. The length of the secondary catheter region protruding beyond the first catheter is adjustable with respect to the dimension of the ischemic tissue region. In the working position the secondary balloon is dilated to define two separate zones for fluid reperfusion: a proximal one between primary and secondary balloons, and a distal one distal to the secondary balloon. By dilating at least the secondary balloon using usually a fluid which fills the secondary balloon with a pressure of more than 8 bar, the secondary balloon directs radially inward compression forces directly onto the secondary balloon catheter so that a standard guidewire lumen inside the secondary balloon catheter will be compressed when there is no reinforcement for ensuring irrigation flow.

Therefore, a high radial strength of the irrigation lumen is required while maintaining flexibility. Therefore a means of radial reinforcement is provided at least in an axial region of the secondary balloon catheter along which the secondary balloon is disposed.

Preferably at least one further means of radial reinforcement is arranged in an axial region of the primary balloon catheter along which the primary balloon is disposed.

The means of radial reinforcement preferably provides at least one ring-shaped structure being integrated in the secondary balloon catheter encircling at least the irrigation lumen to assure an open irrigation lumen at all time during the use of the dual catheter. The ring-shaped structure is preferably monolithically or in form of a braid, since a braided structure allows axial flexibility with an almost constant ring-diameter.

The reinforcement is made using suitable materials, for example a braid with stainless steel, nitinol or other metal wires in a pattern 2 over 2 with preferably 16 wires.

In case of using the dual catheter arrangement with MR-imaging technology, the means of reinforcement can also be out of MR compatible material like PEEK or LCP or glass fibers.

After having placed the dual catheter arrangement in the working position the distal ischemic myocardial zone is reperfused by a controlled release of a liquid which preferably is an oxygenated physiological perfusion solution through the irrigation lumen of the secondary balloon catheter until natural electrical excitability is restored in this distal myocardial zone. By reperfusing the distal ischemic zone in a first step only, PVET cannot give rise to uncontrolled re-entrant excitation as the proximal ischemic myocardial zone is left inexcitable, thus acting as a shield-region of electrical blocker-entry excitation, conceptually similar to scars in ablation therapy.

Upon subsequent complete reperfusion, which is performed in a timely manner following recovery of the distal zone by deflating the secondary balloon and controlled release of perfusion solution through one of the lumens of the primary balloon catheter, PVET in the proximal myocardial zone may be present, but now the path length and hence the associated time-delay in electrical activation are too short to give rise to re-entrant excitation. Thus by dividing reperfusion process into two spatially and temporally separated steps, the arrhythmogenic PVET mechanisms present upon standard reperfusion are diffused and become functionally silent. Subsequently the heart will fully recover without the occurrence of PVET-based reentrant arrhythmias.

In an alternative use of the same embodiment, the proximal perfusion zone may be irrigated with oxygenated cardioplegic solution through one of the lumens of the primary balloon catheter at the same time or during a partially overlapping time-period as perfusion of the distal zone using physiological perfusion solution applied through the irrigation lumen of the secondary catheter is conducted. This restores nutrients and removes waste products in the tissue fed by the proximal perfusion zone, while maintaining inexcitability in the tissue shield-region.

In a preferred embodiment of the dual catheter arrangement an electrode arrangement is attached at the distal end of at least at one of the primary and secondary balloon catheters, which is designed to detect recovery of electrical activity of the cardiac tissue surrounding the distal ends of the primary and secondary balloon catheter respectively.

For example, in order to determine whether natural electrical excitability is reestablished in the distal myocardial zone after a certain period of perfusion, at least one electrode pair is attached to the distal tip of the secondary balloon catheter, which is preferably galvanically connected to an external - and during use extracorporeal - measuring unit, to allow assessment of electrical activity in the distal myocardial zone. For the same purpose the primary balloon catheter has an electrode arrangement at its distal region of the same kind.

After completion of the first step of the SHIELD-EP method, the secondary balloon is deflated and the secondary balloon catheter may or may not remain in place while perfusion solution is delivered to the proximal and distal myocardial zone through one of the lumens of the primary balloon catheter. To more closely monitor the recovery process of electrical excitability within the proximal myocardial zone, a preferred embodiment features a further electrode arrangement proximal to the secondary balloon catheter at least in regions along its longitudinal extent. The further electrode arrangement, which preferably provides at least two, for example annular, electrodes arranged at a distance from one another along the outer circumference of the secondary primary balloon catheter, which are preferably galvanically connected to an external - and during use extracorporeal - measuring unit, designed to detect an electrical activity in the myocardium surrounding the secondary balloon catheter. For the same or a similar purpose, the primary balloon catheter preferably also has a further similar electrode arrangement along its outer circumference proximally to the balloon.

The secondary balloon catheter provides a catheter length which is greater than the length of the primary balloon catheter, so that, in the working position, the secondary balloon catheter extends distally past the primary balloon catheter by a length which normally will not exceed 10 cm for cardiac applications. Depending on the anatomical proportions of a coronary ischemic myocardial area to be treated, it is thus possible to adjust the distance between the primary and secondary balloon variably between a few millimeters and multiple centimeters. So the design of the dual balloon catheter arrangement can be scaled up to other organ systems, like kidney.

In addition, the secondary balloon catheter has an outer diameter that is slightly less than the inner diameter of at least one of the lumens of the primary balloon catheter into which the secondary balloon catheter is slid.

Preferably, but not necessarily, the secondary balloon catheter is completely removable from and re-insertable into the primary balloon catheter, i.e., this lumen can be used in place of the secondary catheter with any other medical instrument of appropriate dimension, for example, in the form of a guidewire, an ablation-, aspiration- or resection-catheter, to name a few.

Both the irrigation channel of the secondary balloon catheter and the at least one lumen of the primary balloon catheter that is not used by the secondary balloon catheter or other medical instrument have a connecting flange proximally for detachable, fluid-tight connection to an irrigation fluid source.

Another embodiment provides a fixed arrangement between the primary and secondary balloon catheter such that the secondary balloon catheter is arranged with a fixed position relative to the primary balloon catheter which corresponds to the working position, in which the secondary balloon catheter continues completely through the lumen of the primary balloon catheter and extends distally beyond the primary balloon catheter. In such a case the primary balloon catheter doesn't require a lumen through which the secondary balloon catheter is inserted slidably, but the primary and secondary balloon catheter are firmly connected, preferably monolithically. In this case, it is advantageous in practical use to have a variety of different dual catheter arrangements, each with fixed distances between the primary and secondary balloon, ranging from a few millimeters to 10 cm. Although such a monolithically catheter arrangement according to the invention could not be described as a typical dual catheter arrangement, since the arrangement can't be divided into two separate catheters, but the inseparability does not change the functionality of the catheter arrangement which can rather be considered as a dual-balloon and dual-flow assembly, which makes it possible to use or perform the SHIELD-EP method in the following way.

The inventive dual catheter arrangement is part of an interventional cardiology system for performing the SHIELD-EP method, which is composed of the following components:
- inflation and deflation units connected fluid-tightly with the lumens for inflation and deflation of the primary and of the secondary balloons,
- a flushing source unit connected fluid-tightly with one of the at least two lumens of the primary balloon catheter and with the irrigation lumen of the secondary balloon catheter, and
- a control unit for controlling the inflation and deflation unit and the flushing source unit in the following manner:
   a) inflating the primary balloon by the inflation and deflation unit,
   b) during or after placing the secondary balloon catheter in the working position, inflating the secondary balloon catheter by the inflation and deflation unit,
   c) controlled release of liquid through the irrigation lumen of the secondary balloon catheter by the flushing source unit until reaching an abort criterion, potentially combined for part of all of the perfusion period with controlled release of a different (e.g. cardioplegic) liquid through the irrigation lumen of the primary balloon catheter by the flushing source,
   d) deflating the secondary balloon by the inflation and deflation unit,
   e) controlled release of a liquid, which may or may not be identical to one of the aforementioned ones, through one of the lumens of the primary balloon catheter by the flushing source unit until reaching the abort criterion, and
   f) deflating the primary balloon by the inflation and deflation unit.

In deviation from the preferred above chronological sequence of steps a) to f), step d) can also be carried out together with the above step f), i.e. deflating of the secondary balloon by the inflation and deflation unit can be carried out during or after the controlled release of liquid through one of the lumens of the primary balloon catheter by the flushing source unit until reaching the abort criterion and together with deflating the primary balloon by the inflation and deflation unit. In addition to a mere flushing in the sense of an exclusive inflow of liquid through one of the lumens of the primary balloon catheter according to the above explained process step e), a further development of the dual catheter provides for the possibility of a suction of liquid from the area between the first and second balloon, so that a circulation can be created during the perfusion of the area between primary and secondary balloon.

Basically there are three options for establishing a circulation: a) Deflate primary balloon and suck via one lumen extending throughout the primary balloon catheter, b) Deflate secondary balloon and perfuse via irrigation lumen of the secondary balloon catheter and suck via one lumen extending throughout the primary balloon catheter to get counterflow and c) Add a secondary suction lumen to the primary balloon catheter to wash while keeping both balloons inflated.

Such a further formed dual catheter is explained below with reference to the figures.

The main criterion of the SHIELD-EP method is the independently controlled timing, location, and composition of two-zone reperfusion, so that the distal ischemic myocardial zone recovers electrical excitability earlier than the proximal ischemic myocardial zone. After deflation of the primary and secondary balloon, the dual catheter arrangement can be removed.

In a preferred embodiment the interventional cardiology system further provides an electrical measurement unit which is connected or connectable at least with the electrode arrangements which are attached at least at the distal ends of the primary and secondary balloon catheter, each delivering measuring signals, which are used in the control unit as a basis for determining the abort criterion. In those cases in which the primary and/or secondary balloon catheter have further electrodes distributed at least in areas along the primary and/or secondary balloon catheter sleeves, these electrodes are individually connected or connectable with the electrical measurement unit for delivering measuring signals, which may be used in the control unit as a basis for determining the abort criterion also. The measuring signals obtained by means of the electrode arrangements reflect the electrical activity of the myocardium immediately adjacent to the primary and/or secondary balloon catheter, which is used to assess the state of local cardiac electrical excitability. The measured signals are compared with the electrical excitability of healthy myocardial tissue as part of a target/actual value comparison. A predefinable minimum approximation to this target value serves as an abort criterion.

### BRIEF DESCRIPTION OF THE DRAWINGS

The invention will be described below in an exemplary manner by way of embodiments with reference to the figures, without any limitation of the general inventive concept. Here:
- Fig. 1: A schematic representation of the dual catheter arrangement in combination with inflation and deflation unit, flushing source unit, electrical measurement unit and control unit,
- Fig. 2: sequential steps for performing SHIELD-EP method using the dual catheter arrangement and
- Fig. 3a-c: Cross-section, distal view and perspective view of the secondary balloon catheter,
- Fig. 4.: Cross-section of the primary balloon catheter and
- Fig. 5a, b: Alternative fixed-distance dual catheter arrangements.

### DETAILED DESCRIPTION OF THE INVENTION

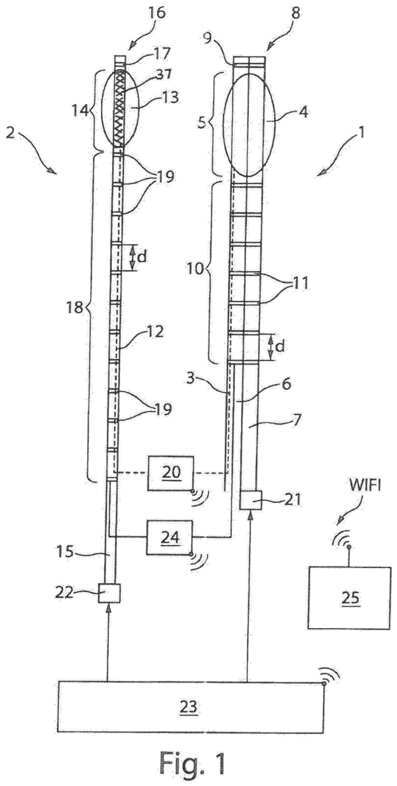

Figure 1 illustrates a system for reperfusion of an ischemic tissue region in a coronary vessel comprising a dual catheter arrangement, which consists of a primary balloon catheter 1 and a secondary balloon catheter 2.

The primary balloon catheter 1 provides a lumen 3 for inflation and deflation of a primary balloon 4 positioned at a distal region 5 of the primary balloon catheter 1, and at least two separate lumens 6, 7 both extending throughout the primary balloon catheter 1 and open at a distal end 8 of the primary balloon catheter 1. The primary balloon catheter 1 further provides at its distal end 8 a first electrode arrangement 9, preferably in the form of one or multiple ring electrodes encircling the distal end 8, and a second electrode arrangement 10, which is attached proximally to the primary balloon 4 comprising at least two electrodes 11, preferably a multitude of electrodes 11, being arranged at the outer periphery of the primary balloon catheter 1 with a distance d from each other, which isn't necessarily constant but preferably.

The secondary balloon catheter 2 has a lumen 12 for inflation and deflation of a secondary balloon 13 disposed at the distal region 14 of the secondary balloon catheter 2 and an irrigation lumen 15 opening at the distal end 16 of the secondary balloon catheter 2. The secondary balloon catheter 2 is dimensioned and formed such, that the secondary balloon catheter 2 is insertable into one of the lumens 6 of the primary balloon catheter 1 in a slidable manner, when the secondary balloon 13 is deflated. The secondary balloon catheter 2 further provides at its distal end 16 a third electrode arrangement 17, preferably in the form of one or multiple ring electrodes encircling the distal end 16, and a fourth electrode arrangement 18, which is attached proximally to the secondary balloon 13 comprising at least two electrodes 19, preferably a multitude of electrodes 19, being arranged at the outer periphery of the secondary balloon catheter 2 with a distance d from each other, which isn't necessarily constant but preferably.

By dilating at least the secondary balloon 13 using usually a fluid which fills the secondary balloon 13 with a pressure of more than 8 bar, the secondary balloon 13 directs radially inward compression forces directly onto the secondary balloon catheter 2 so that the irrigation lumen 15 inside the secondary balloon catheter 2 will be compressed when there is no reinforcement for ensuring irrigation flow. Therefore, a high radial strength of the irrigation lumen 15 is required, while maintaining axial flexibility. Therefore a means of radial reinforcement 37 is provided at least in an axial region of the secondary balloon catheter 2 along which the secondary balloon 13 is disposed.

The means of radial reinforcement 37 is made using suitable materials, for example a braid with stainless steel, nitinol or other metal wires in a pattern 2 over 2 with preferably 16 wires. If the dual catheter arrangement should be used with MR-imaging technology, the means of radial reinforcement 37 can also be out of MR compatible material like PEEK or LCP or glass fibers. The means of radial reinforcement 37 provides at least one ring-shaped structure being integrated in the secondary balloon catheter 2 encircling at least the irrigation lumen 15 to assure an open irrigation lumen 15 at all time during the use of the dual catheter arrangement. The ring-shaped structure is preferably monolithically or in form of a braid, since a braided structure allows axial flexibility with an almost constant ring-diameter. The ring-shaped structure provides a radial outer layer made of a polymer that can be connected, by e.g. a welding process, with the secondary balloon 13. Common materials to be used are PEBAX, Nylon 11 or Nylon 12. The preferable dimensions of the inner lumen of the ring-shaped structure are: Inner diameter: 0.45 mm to 0.50 mm and outer diameter: 0.65 mm to 0.75 mm.

The lumens 3, 12 for inflation and deflation of the primary and secondary balloon 4, 13 are fluid-tightly connected or connectable with an inflation and deflation unit 20. The irrigation lumen 15 of the secondary balloon catheter 2 and the lumen 7 which is not used for the secondary balloon catheter 2 provide at its proximal ends a connecting flange 21, 22 each for detachable fluid-tight connection to a flushing source 23. Further the first, second, third and fourth electrode arrangements 9, 10, 17, 18 on the primary and secondary balloon catheters respectively are each connected galvanically with an electrical measurement unit 24. The inflation and deflation unit 20, the flushing source unit 23, and the electrical measurement unit 24 are wired or wirelessly connected to a control unit 25 that controls the operation and activity of all units so that the operation of the units and the dual catheter assembly associated with them can be operated semi- or fully automatically. In figure 1 a radio based data exchange WIFI between the units 20, 23, 24 and 25 is illustrated.

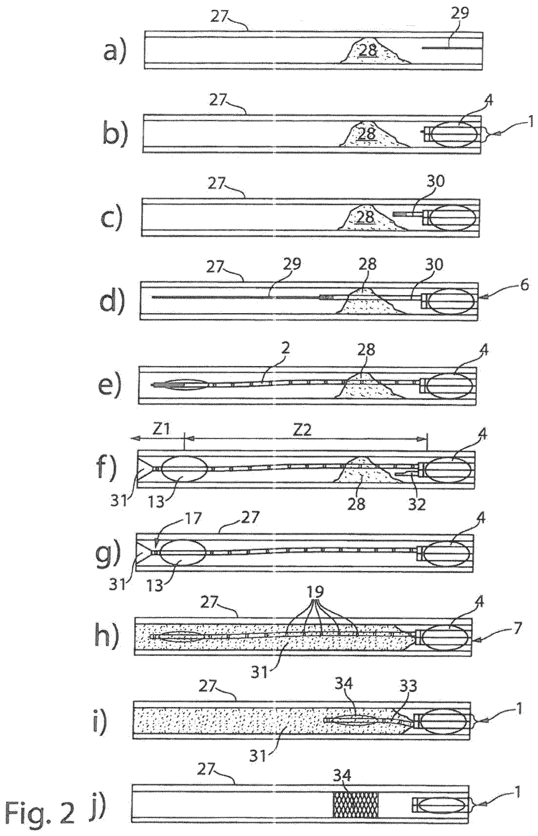

In figures 2a-j sequential steps for performing SHIELD-EP method using the dual catheter arrangement are illustrated.

Fig. 2a shows a cardiac blood vessel 27, which is occluded by a thrombus 28. Assume that the tissue area A1 to the right of the thrombus 28 is perfused, whereas the tissue area A2 to the left of the thrombus 28 is ischemic.

After identifying the position of the thrombus 28, for example by angiography, a guidewire 29 is inserted into the cardiac blood vessel 27 lumen, for example via the femoral artery, and moved next to the thrombus 28, for example under X-ray guidance.

In a next step the primary balloon catheter 1 is passed over the guidewire 29 into the blood vessel 27 and positioned in front, i.e. proximally, of the thrombus 28, see figure 2b. For this purpose, the lumen 6 of the primary balloon catheter 1 passes over guide wire 29. The primary balloon 4 is then inflated, ensuring the blood vessel 27 remains blocked and unharmed and the primary catheter remains firmly positioned.

After inflating the primary balloon 4 a thrombus crossing catheter 30 is then introduced in the case of a total occlusion of the blood vessel 27 by the thrombus 28, see figure 2c. In case of an incomplete occlusion this step could be skipped.

The thrombus crossing catheter 30 passed through a lumen 6 of the primary balloon catheter 1 over the guidewire 29, see figure 2d. Once the thrombus 28 is crossed the guidewire 29 is then further advanced distally while the thrombus crossing catheter 30 is removed proximally and in its place the secondary balloon catheter 2 is introduced into the lumen 6 of the primary balloon catheter 1, see figure 2e.

The secondary balloon 13 then is inflated in a working position, in which the secondary balloon 13 is within the ischemic area, distally of the occluded vessel area, and further divides this ischemic area in a distal ischemic myocardial zone Z1, distally to the secondary balloon 13, and a proximal ischemic myocardial zone Z2, which is between the primary and secondary balloon 4, 13, see figure 2f.

Once in the working position perfusion of the distal ischemic zone Z1 is started, by releasing of perfusion solution 31 through the irrigation lumen of the secondary balloon catheter 2. At the same time an aspiration catheter 32 may be used in the other lumen 6 of the primary balloon catheter 1 to remove the thrombus 28, see figure 2f again.

The aspiration catheter is removed upon removal of the thrombus 28, while perfusion is continued until the distal zone Z1 is once again excitable as measured using the distal electrode 17 of the secondary balloon catheter 2, see figure 2g.

The secondary balloon 13 is then deflated, the secondary balloon catheter 2 can remain in place and perfusion of the proximal myocardial zone Z2 is started, see figure 2h, by a controlled release of perfusion solution through the lumen 7 of the primary balloon catheter 1 until the proximal zone Z2 is once again excitable as measured using the electrodes 19 of the secondary balloon catheter 2 and the distal electrode arrangement 9 of the primary balloon catheter 1, see figure 2h. The perfusion solution released through the lumen 7 of the primary balloon catheter 1 also reaches the distal area Z1, since the secondary balloon is deflated and thus no longer represents a fluid barrier.

During or after perfusion of the proximal myocardial zone Z2 a stent catheter 33 having a stent 34 can be introduced through a lumen of the primary balloon catheter 1, see figure 2i.

After the stent 34 is placed in the region of the removed thrombus, the remaining primary balloon 4 is deflated and subsequently all catheters are removed, see figure 2j.

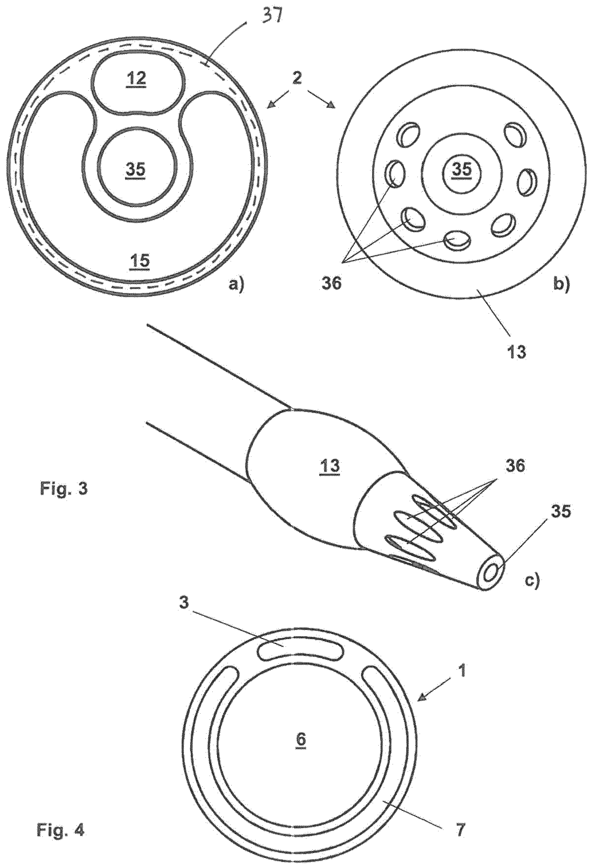

To facilitate insertion and navigation of the secondary balloon catheter 2, it provides a central lumen 35 for the guidewire 29 that extends axially completely through the secondary balloon catheter 2, see Figures 3a, b, c.

Figure 3a shows a cross-sectional view of an alternative embodiment of the secondary balloon catheter 2, Figure 3b shows an axial view of the distal end 16 of the secondary balloon catheter 2, and Figure 3c shows a perspective view of the distal end region 14 of the secondary balloon catheter 2.

The guidewire lumen 35 has a diameter that is slightly oversized to match the diameter of a typical guidewire to ensure that the guidewire can easily slide through the lumen but, on the other hand, no or no appreciable amount of fluid, particularly perfusion solution, can enter the guidewire lumen in the proximal direction. Depending on the choice of a guidewire, the diameter of the guidewire lumen is about 300 to 1000 µm.

The lumen for irrigation 15 and the lumen for inflating and deflating the secondary balloon 13 are arranged radially around the central guide wire lumen 35. In order to enable the most effective and at the same time tissue-friendly irrigation with perfusion solution, the lumen for irrigation 15 has the largest possible cross-section, which encompasses the central guide wire lumen 35 in a kidney-shaped manner, see Figure 3a. The perfusion solution exits the secondary balloon catheter 2 via a plurality of outlet openings 36 at the distal end of the second balloon catheter 2. For this purpose, the outlet openings 36 are preferably arranged radially around the central distal opening of the guide wire lumen 35, see Figure 3b. Due to the multiplicity and spatial arrangement of the outlet openings 36 around the central guidewire lumen 35, the perfusion solution can emerge from the distal end of the second balloon catheter 2 with both the lowest possible flow speed and a spatially adjustable flow divergence, whereby the distal tissue can be perfused gently and uniformly over large areas. The flow divergence of the emerging perfusion solution can be optimized by suitable selection of the axial orientations of the individual outlet openings 36.

Additionally a means of radial reinforcement 37 is integrated into the secondary balloon catheter 2 at least in an axial region of the secondary balloon catheter 2 along which the secondary balloon 13 is disposed.

Proximal to the outlet openings 36 is the secondary balloon 13, which is fluidically connected via the lumen 13 for inflating and deflating.

Figure 4 shows a cross-section through the primary balloon catheter 1, which provides a preferably central lumen 6 for passing through, for example, the secondary balloon catheter 2, a lumen 7 for the perfusion solution and a lumen 3 for inflating and deflating the first balloon 4. In this case, the lumen 3 for inflating and deflating the first balloon 4 and the lumen 7 for the perfusion solution are arranged radially around the central lumen 6 for passing through, for example, the secondary balloon catheter 2.

The proposed stepwise reperfusion technique SHIELD-EP can comprise the following steps:
A method of spatially and temporally controlled reperfusion of two distinct zones an ischemic tissue region with defined solutions (type, flow rate and pressure, temperature, oxygenation, etc.) in a coronary vessel caused by an occlusion along a blood-carrying vessel, comprising the steps of:
- locating the occlusion and placing a primary balloon proximal to the occlusion within the vessel such that tissue region adjacent to the vessel is perfused with blood proximally to the primary balloon and is ischemic distally,
- placing a secondary balloon distal to the occlusion within the vessel in the ischemic tissue region, which together with the first balloon limit an interstitial space, further referred as proximal myocardial zone Z2, and further limits a distal myocardial zone Z1 distal to the secondary balloon,
- perfusing the distal myocardial zone Z1 with a perfusion solution, while proximal myocardial zone Z2 remains untreated and ischemic until reaching an abort criterion or subjected to separately controlled perfusion with a suitable solution that keep proximal tissue non-excitable,
- perfusing at least the proximal myocardial zone Z2 with a perfusion solution until reaching an abort criterion
- removing the primary and secondary balloon from the vessel.

The time gap between both perfusing steps can be specified individually. The distance between the primary and secondary balloons can be specified individually as well. The flow rates and perfusion pressures for the perfusion solution in each perfusion line is controlled independently. Also it is possible release a selected liquid of at least two different liquids through each of the lumens of both catheters.

Further it is of advantage to remove the occlusion after placing at least the primary balloon, preferably after placing both, the primary and the secondary balloon. After removing all the balloons it may be of advantage to implement a stent at the location of the removed occlusion for avoiding any local later constriction.

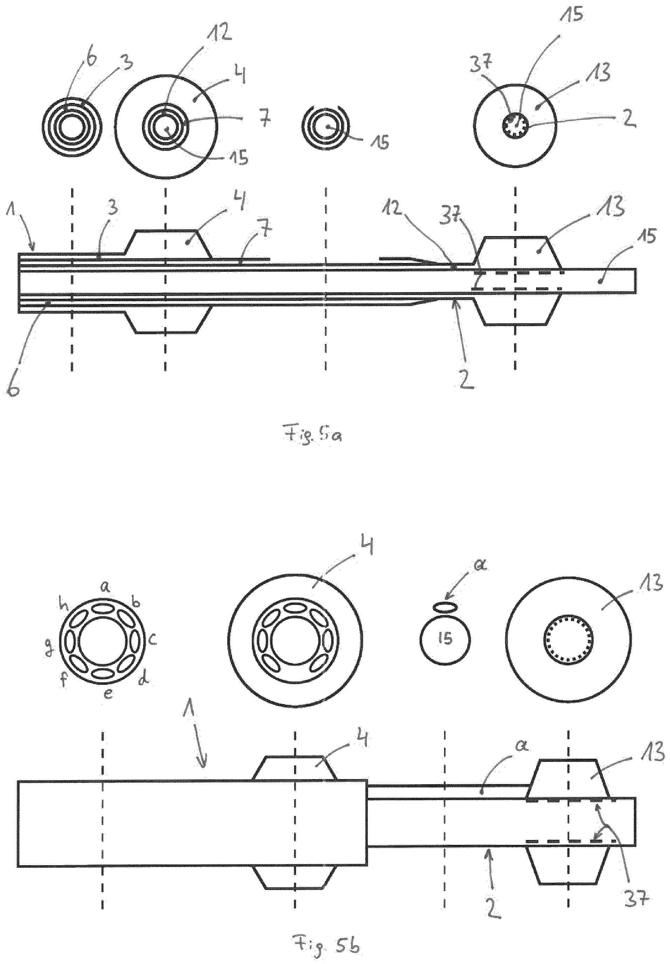

Figures 5 a and b show longitudinal sections through alternative embodiments of a catheter arrangement with a primary balloon catheter 1 and a secondary balloon catheter 2 being combined in one piece and having a fixed position to each other. Along each longitudinal sections cross sections through the catheter arrangement are illustrated at positions marked by dashed lines.

In case of embodiment shown in Figure 5a the primary balloon catheter 1 encircles a) the lumen for inflation and deflation 3 which is fluid tight connected with the primary balloon 4, b) the irrigation lumen 7 opening between the primary balloon 4 and the secondary balloon 13 and c) the lumen 6 along which the secondary balloon catheter 2 is arranged encircling the in- and deflation lumen 12 for the secondary balloon 13 and at least the irrigation lumen 15. A means of radial reinforcement 37 is arranged radially inwardly to the secondary balloon 13 along the secondary balloon catheter 2 which encloses at least the irrigation lumen 15.

The graphic representation of further lumens, which have been described in connection with the above embodiment examples, is omitted here merely for reasons of better visual representability.

The primary and secondary balloon 4, 13 are made of a balloon material preferable PEBAX, Nylon 11 or Nylon 12, a blend of these materials and/or a multi-layer extrusion, where these materials and or blends of these materials are used.

The secondary balloon 13 has a diameter range of 2mm up to 6mm with a preferable compliance of 10% between Nominal Pressure and Rated Burst Pressure. The primary balloon 4 has a diameter range of 2.5 mm up to 6 mm with a preferable compliance of 10% between Nominal Pressure and Rated Burst Pressure. The length of the balloons is preferably 1 mm up to 3 mm.

The means of radial reinforcement 37 can be built using a 2-layer extrusion that is reinforced with a braided structure. The reinforced structure can be made like the braided structure as described using PEEK, LCP or glass for braiding or nitinol.

The irrigation lumen 7 is a single lumen normally so that perfusion/irrigation can be performed in one direction, i.e. towards distal direction, only. It is also possible to modify the one channel irrigation lumen 7 in a multi lumen tube so that a circulation can be created during the irrigation/perfusion of the area between primary and secondary balloon 4, 13. Therefore at the proximal end of the irrigation lumen 7 a further additional side port will be required. In such case, one side port will be connected to the in-flow of medium to be perfused, the other side port is connected to the out-flow being proximally connected with a suction source.

Figure 5 b shows an alternative embodiment of the dual catheter arrangement with a primary balloon catheter 1 having an eight lumen tubing a-h, preferably produced by extrusion out of PEEK. The PEEK is preferable in amorphous condition. The eight lumens a-h are arranged axially parallel to each other in circumferential direction side by side in the wall of the primary balloon catheter 1 as illustrated in left the cross section of Figure 5b. From the eight lumens ah, lumen a) is used as inflation lumen for the secondary balloon 13 and lumen e) is used for the inflation of the primary balloon 4. The other six lumens b,c,d,f,g,h are used for the perfusion/irrigation of the region between the primary balloon 4 and secondary balloon 13.

A means of radial reinforcement 37 is arranged radially inwardly to the secondary balloon 13 along the secondary balloon catheter 2 which encloses at least the irrigation lumen 15, while the innermost lumen serves to perfuse the myocardium distal to the secondary balloon.

Of course, the means of radial reinforcement 37 can additionally be arranged radially inward of the primary balloon 4 along the primary balloon catheter as in case of all before disclosed embodiments.

Further preferred embodiments in connection with the inventive dual catheter arrangement provide for the following features:
- On the dual catheter arrangement radio opaque markers can be applied so that the position of the primary and/or secondary balloon catheter 1, 2 and the primary and secondary balloon 4, 13 are visible under X-ray.
- When the dual catheter arrangement respectively the dual-balloon / dual-flow catheter arrangement is designed to be used with MR-Imaging than the markers will be made out of material so that the position of the catheter is visible with the MR-Imaging. Further the primary and secondary balloon 4, 13 can have a layer of special material that has iron particles compounded into the balloon material.
- The primary balloon 4 can be a balloon for stent delivery and can carry a stent that can be implanted into the artery during the interventional procedure.
- The third electrode arrangement 17 of secondary balloon catheter 2 and/or the first electrode arrangement 9 of primary balloon catheter 1 provides bi-polar electrodes so that an electrical stimulation can be applied.
- The perfusion solution that general is used for distal and proximal perfusion is a saline solutions which is electrically conductive and which can be used to transmit electrical power for stimulation. Stimulation electrodes, which are placed proximally in the area of the liquid connectors, are used to contact the perfusion solution electrically.
- Bipolar electrode pairs can be positioned on either side of the primary and/or secondary balloon 4, 13 to assess local tissue activation.
- To avoid particles floating into more distal parts of the coronary vasculature, the two fluid streams can be used as a rinsing system at the end of the interventional procedure. This may involve deflating the primary balloon 4 while keeping the secondary balloon 13 inflated, with aspiration of blood from the proximal perfusion lumen 7. Alternatively, it may involve deflating the secondary balloon 13 while keeping the primary balloon 4 inflated, flowed by saline infusion into the irrigation lumen 15, and aspiration from proximal perfusion lumen 7. Fluid should be injected through the irrigation lumen 15 during deflation of the secondary balloon 13 while aspiration is begun via the proximal perfusion lumen 7. This may be combined with applications of treatments, such as thrombolytics. In the embodiment shown in Fig. 5b, it is also possibly to arrange flushing of the inter-balloon vessel segment by using some of the 6 primary fluid lines for inflow, and the others for suction.

### List of reference signs

- 1: primary balloon catheter
- 2: secondary balloon catheter
- 3: lumen for inflation and deflation
- 4: primary balloon
- 5: distal region of primary balloon catheter
- 6: lumen of primary balloon catheter
- 7: lumen of primary balloon catheter
- 8: distal end of primary balloon catheter
- 9: first electrode arrangement of primary balloon catheter
- 10: second electrode arrangement of primary balloon catheter
- 11: electrodes of primary balloon catheter
- 12: lumen
- 13: secondary balloon
- 14: distal region of secondary balloon catheter
- 15: irrigation lumen
- 16: distal end of secondary balloon catheter
- 17: third electrode arrangement of secondary balloon catheter
- 18: fourth electrode arrangement of secondary balloon catheter
- 19: electrodes of secondary balloon catheter
- 20: inflation and deflation unit
- 21: connecting flange
- 22: connecting flange
- 23: flushing source unit
- 24: electrical measurement unit
- 25: control unit
- 26: thrombus
- 27: cardiac blood vessel
- 28: thrombus
- 29: guidewire
- 30: thrombus crossing catheter
- 31: perfusion solution
- 32: aspiration catheter
- 33: stent catheter
- 34: stent
- 35: lumen for guidewire
- 36: outlet opening
- 37: means of radial reinforcement
- Z1: distal ischemic zone
- Z2: proximal ischemic myocardial zone

## Claims

1. A dual catheter arrangement for reperfusion of an ischemic tissue region via a coronary vessel, comprising a primary balloon catheter (1) having a lumen (3) for inflation and deflation of a primary balloon (4) disposed at a distal region (5) of the primary balloon catheter (1) and a lumen (6) extending throughout the primary balloon catheter (1) and open at a distal end (8) of the primary balloon catheter (1), and a secondary balloon catheter (2) having a lumen (12) for inflation and deflation of a secondary balloon (13) being disposed at a distal region (14) of the secondary balloon catheter (2), said secondary balloon catheter (2) is arranged through the lumen (6) of the primary balloon catheter (1), so that the secondary balloon catheter (2) continues completely through the lumen (6) of the primary balloon catheter (1) and extends distally beyond the primary balloon catheter (1) **wherein** the primary balloon catheter (1) has at least a further separate lumen (7) extending throughout the primary balloon catheter (1) and open at a distal end (8) of the primary balloon catheter (1) having a connecting flange (21) proximally for detachable, fluid-tight connection to a flushing source unit (23) and open at the distal end (8) of the primary balloon catheter (1), and the secondary balloon catheter (2) has an irrigation lumen (15) having a connecting flange proximally for detachable, fluid-tight connection to a flushing source unit (23) and an opening at a distal end (16) of the secondary balloon catheter (2), said secondary balloon catheter (2) provides at least in an axial region along which the secondary balloon (13) is disposed a means of radial reinforcement.

2. The dual catheter arrangement according to claim 1,
wherein a first electrode arrangement (9) is attached at least at the distal end (8) of the primary balloon catheter (1) being designed to detect an electrical activity of a tissue surrounding the distal end (8) of the first balloon catheter (1).

3. The dual catheter arrangement according to claim 1 or 2,
wherein a second electrode arrangement (10) is attached proximally to the primary balloon (4) at least in areas along the primary balloon catheter (1), being designed to detect an electrical activity of a tissue surrounding the primary balloon catheter (1).

4. The dual catheter arrangement according to one of the claims 1 to 3,
wherein a third electrode arrangement (17) is attached at the distal end (8) of the secondary balloon catheter (2), which is designed to detect an electrical activity of a tissue surrounding the distal end (16) of the secondary balloon catheter (2).

5. The dual catheter arrangement according to one of the claims 1 to 4,
wherein a fourth electrode arrangement (18) is attached proximally to the secondary balloon (13) at least in areas along the secondary balloon catheter (2), being designed to detect an electrical activity of a tissue surrounding the second balloon catheter (2).

6. The dual catheter arrangement according to claim 3 or 5,
wherein the second electrode arrangement (10) provides at least two electrodes (11) being arranged at a distance from each other along an outer periphery of the primary balloon catheter (1) and
wherein the fourth electrode arrangement (18) provides at least two electrodes (19) being arranged at a distance from each other along an outer periphery of the secondary catheter (2).

7. The dual catheter arrangement according to one of the claims 1 to 6,
wherein the secondary balloon catheter (2) provides a catheter length which is greater than a length of the primary balloon catheter (1), so that, the secondary balloon catheter (2) towers the primary balloon catheter (2) distalwards by a length of up to 10 cm.

8. The dual catheter according to one of the claims 1 to 7,
wherein the secondary balloon catheter (2) has a lumen for a guidewire which completely protrudes axially through the secondary balloon catheter and opening at the distal end (16) of the secondary balloon catheter (2).

9. The dual catheter according to claim 8,
wherein the lumen for the guidewire extends centrally through the second balloon catheter and the irrigation lumen (15) and the lumen for inflation and deflation of the secondary balloon (13) are arranged radially around the lumen for the guidewire.

10. The dual catheter according to claim 8 or 9,
wherein the lumen for a guidewire has a constant diameter ranging between 300 and 1000 µm.

11. The dual catheter according to one of the claims 1 to 10,
wherein the secondary balloon catheter (2) is arranged to be slidable within the lumen (6) of the primary balloon catheter (1), to achieve a working position in which the secondary balloon catheter (2) continues completely through the lumen (6) of the primary balloon catheter (1) and extends distally beyond the primary balloon catheter (1).

12. The dual catheter according to one of the claims 1 to 10,
wherein the secondary balloon catheter (2) is arranged within the lumen (6) of the primary balloon catheter (1) in a fixed position in which the secondary balloon catheter (2) continues completely through the lumen (6) of the primary balloon catheter (1) and extends distally beyond the primary balloon catheter (1).

13. The dual catheter according to one of the claims 1 to 12,
wherein the means of radial reinforcement (37) provides at least one ring-shaped structure being integrated in the secondary balloon catheter (2) encircling at least the irrigation lumen (15).

14. The dual catheter according to claim 13,
wherein the ring-shaped structure is monolithically or in form of a braid.

15. The dual catheter according to claim 13 or 14,
wherein the ring-shaped structure is made of at least one of the following materials: stainless steel, nitinol, PEEK, LCP, glass.

16. The dual catheter according to one of the claims 1 to 15,
wherein the primary balloon catheter (1) provides at least one suction lumen having an opening at it's distal end.

17. System for reperfusion of an ischemic tissue region via a coronary vessel comprising the dual catheter arrangement of one of the claims 1 to 16, further comprising
- an inflation and deflation unit (20) connected fluid-tightly with the lumen (3, 12) for inflation and deflation of the primary and secondary balloon (1, 2),
- a flushing source unit (23) connected fluid-tightly with the further separate lumen (7) of the primary balloon catheter (1) and with the irrigation lumen (15) of the secondary balloon catheter (2), and
- a control unit (25) configured to control the inflation and deflation unit (20) and the flushing source unit (23) in the following manner:
- inflating the primary balloon (4) by the inflation and deflation unit (20),
- during or after placing the secondary balloon catheter (2) in the working position, inflating the secondary balloon (13) by the inflation and deflation unit (20),
- controlled release of liquid through the irrigation lumen (15) of the secondary balloon catheter (2) by the flushing source unit (23) until reaching an abort criterion,
- deflating the secondary balloon (13) by the inflation and deflation unit (20),
- controlled release of liquid through one of the lumens (6 or 7) of the primary balloon catheter (1) by the flushing source unit (23) until reaching an abort criterion, and
- deflating the primary balloon (4) by the inflation and deflation unit (20).

18. System according to claim 17 with the dual catheter arrangement according to claims 2 and 4,
wherein an electrical measurement unit (24) is connected at least with the first and third electrode arrangement (9, 17) delivering measuring signals, which are used in the control unit (25) as a basis for determining the abort criterion.

19. System according to claim 18 with the dual catheter arrangement according to claims 3 and 5,
wherein the electrical measurement unit (24) is connected at least with the second and fourth electrode arrangement (10, 18) delivering measuring signals, which are used in the control unit (25) as a basis for determining the abort criterion.

20. System according to one of the claims 17 to 19,
wherein the control unit (25) is configured to control the flushing source unit (23) such that at least one of the at least two lumens (6 or 7) of the primary balloon catheter (1) and the irrigation lumen (15) of the secondary balloon catheter (2) can be flushed independently with control over time, pressure, flow, temperature and /or type of liquid.

21. System according to one of the claims 17 to 20,
wherein the control unit (25) is configured to start the controlled release of liquid through one of the lumens (6 or 7) of the primary balloon catheter (1) by the flushing source (23) timely before or during deflating the secondary balloon (13) by the inflation and deflation unit (20).

## Patentansprüche

1. Doppelkatheteranordnung zur Reperfusion einer ischämischen Geweberegion über ein Koronargefäß, umfassend einen primären Ballonkatheter (1), der ein Lumen (3) zum Aufblasen und Ablassen eines primären Ballons (4), der an einer distalen Region (5) des primären Ballonkatheters (1) angeordnet ist, und ein Lumen (6), das sich durch den primären Ballonkatheter (1) erstreckt und an einem distalen Ende (8) des primären Ballonkatheters (1) offen ist, aufweist, und einen sekundären Ballonkatheter (2), der ein Lumen (12) zum Aufblasen und Ablassen eines sekundären Ballons (13), der an einer distalen Region (14) des sekundären Ballonkatheters (2) angeordnet ist, aufweist, wobei der sekundäre Ballonkatheter (2) durch das Lumen (6) des primären Ballonkatheters (1) angeordnet ist, so dass sich der sekundäre Ballonkatheter (2) vollständig durch das Lumen (6) des primären Ballonkatheters (1) fortsetzt und sich in distaler Richtung über den primären Ballonkatheter (1) hinaus erstreckt,
wobei der primäre Ballonkatheter (1) zumindest ein weiteres gesondertes Lumen (7) aufweist, das sich durch den primären Ballonkatheter (1) erstreckt und an einem distalen Ende (8) des primären Ballonkatheters (1) offen ist, wobei es einen Verbindungsflansch (21) in proximaler Richtung für eine abnehmbare, fluiddichte Verbindung mit einer Spülungsquelleneinheit (23) und an dem distalen Ende des primären Ballonkatheters (1) offen aufweist, und der sekundäre Ballonkatheter (2) ein Spülungslumen (15) aufweist, das einen Verbindungsflansch (21) in proximaler Richtung für eine abnehmbare, fluiddichte Verbindung mit einer Spülungsquelleneinheit (23) und eine Öffnung an einem distalen Ende (16) des sekundären Ballonkatheters (2) aufweist, wobei der sekundäre Ballonkatheter (2) zumindest in einer axialen Region, entlang dessen der sekundäre Ballon (13) angeordnet ist, ein Mittel zur radialen Verstärkung bereitstellt.

2. Doppelkatheteranordnung nach Anspruch 1,
wobei eine erste Elektrodenanordnung (9) zumindest an dem distalen Ende (8) des primären Ballonkatheters (1) befestigt ist, wobei sie dafür ausgelegt ist, eine elektrische Aktivität eines Gewebes zu erfassen, welches das distale Ende (8) des ersten Ballonkatheters (1) umgibt.

3. Doppelkatheteranordnung nach Anspruch 1 oder 2,
wobei eine zweite Elektrodenanordnung (10) proximal zu dem primären Ballon (4) zumindest in Bereichen entlang des primären Ballonkatheters (1) befestigt ist, wobei sie dafür ausgelegt ist, eine elektrische Aktivität eines Gewebes zu erfassen, das den primären Ballonkatheter (1) umgibt.

4. Doppelkatheteranordnung nach einem der Ansprüche 1 bis 3,
wobei eine dritte Elektrodenanordnung (17) an dem distalen Ende (8) des sekundären Ballonkatheters (2) befestigt ist, die dafür ausgelegt ist, eine elektrische Aktivität eines Gewebes zu erfassen, welches das distale Ende (16) des sekundären Ballonkatheters (2) umgibt.

5. Doppelkatheteranordnung nach einem der Ansprüche 1 bis 4,
wobei eine vierte Elektrodenanordnung (18) proximal zu dem sekundären Ballon (13) zumindest in Bereichen entlang des sekundären Ballonkatheters (2) befestigt ist, wobei sie dafür ausgelegt ist, eine elektrische Aktivität eines Gewebes zu erfassen, das den sekundären Ballonkatheter (2) umgibt.

6. Doppelkatheteranordnung nach Anspruch 3 oder 5,
wobei die zweite Elektrodenanordnung (10) mindestens zwei Elektroden (11) bereitstellt, die in einer Entfernung voneinander entlang eines Außenumfangs des primären Ballonkatheters (1) angeordnet sind, und
wobei die vierte Elektrodenanordnung (18) mindestens zwei Elektroden (19) bereitstellt, die in einer Entfernung voneinander entlang eines Außenumfangs des sekundären Ballonkatheters (2) angeordnet sind.

7. Doppelkatheteranordnung nach einem der Ansprüche 1 bis 6,
wobei der sekundäre Ballonkatheter (2) eine Katheterlänge bereitstellt, die größer ist als eine Länge des primären Ballonkatheters (1), so dass der sekundäre Ballonkatheter (2) den primären Ballonkatheter (1) in distaler Richtung um eine Länge von bis zu 10 cm überragt.

8. Doppelkatheteranordnung nach einem der Ansprüche 1 bis 7,
wobei der sekundäre Ballonkatheter (2) ein Lumen für einen Führungsdraht aufweist, das vollständig in axialer Richtung durch den sekundären Ballonkatheter vorspringt und sich an dem distalen Ende (16) des sekundären Ballonkatheters (2) öffnet.

9. Doppelkatheteranordnung nach Anspruch 8,
wobei sich das Lumen für den Führungsdraht mittig durch den zweiten Ballonkatheter erstreckt und das Spülungslumen (15) und das Lumen zum Aufblasen und Ablassen des sekundären Ballons (13) in radialer Richtung um das Lumen für den Führungsdraht angeordnet sind.

10. Doppelkatheteranordnung nach Anspruch 8 oder 9,
wobei das Lumen für einen Führungsdraht einen konstanten Durchmesser aufweist, der zwischen 300 und 1000 µm beträgt.

11. Doppelkatheteranordnung nach einem der Ansprüche 1 bis 10,
wobei der sekundäre Ballonkatheter (2) so angeordnet ist, dass er innerhalb des Lumens (6) des primären Ballonkatheters (1) verschiebbar ist, um eine Arbeitsposition zu erreichen, in der sich der sekundäre Ballonkatheter (2) vollständig durch das Lumen (6) des primären Ballonkatheters (1) fortsetzt und sich in distaler Richtung über den primären Ballonkatheter (1) hinaus erstreckt.

12. Doppelkatheteranordnung nach einem der Ansprüche 1 bis 10,
wobei der sekundäre Ballonkatheter (2) in einer festen Position innerhalb des Lumens (6) des primären Ballonkatheters (1) angeordnet ist, in der sich der sekundäre Ballonkatheter (2) vollständig durch das Lumen (6) des primären Ballonkatheters (1) fortsetzt und sich in distaler Richtung über den primären Ballonkatheter (1) hinaus erstreckt.

13. Doppelkatheteranordnung nach einem der Ansprüche 1 bis 12,
wobei das Mittel zur radialen Verstärkung (37) mindestens eine ringförmige Struktur bereitstellt, die in dem sekundären Ballonkatheter (2) integriert ist, wobei sie zumindest das Spülungslumen (15) umschließt.

14. Doppelkatheteranordnung nach Anspruch 13,
wobei die ringförmige Struktur monolithisch oder in Form eines Geflechts vorliegt.

15. Doppelkatheteranordnung nach Anspruch 13 oder 14,
wobei die ringförmige Struktur aus mindestens einem von den folgenden Materialien hergestellt ist: rostfreiem Stahl, Nitinol, PEEK, LCP, Glas.

16. Doppelkatheteranordnung nach einem der Ansprüche 1 bis 15,
wobei der primäre Ballonkatheter (1) mindestens ein Absaugungslumen bereitstellt, das an seinem distalen Ende eine Öffnung aufweist.

17. System zur Reperfusion einer ischämischen Geweberegion über ein Koronargefäß, wobei das System die Doppelkatheteranordnung nach einem der Ansprüche 1 bis 16 umfasst, wobei es ferner Folgendes umfasst:
- eine Aufblas- und Ablasseinheit (20), die fluiddicht mit dem Lumen (3, 12) zum Aufblasen und Ablassen des primären und des sekundären Ballonkatheters (1, 2) verbunden ist,
- eine Spülungsquelleneinheit (23), die fluiddicht mit dem weiteren gesonderten Lumen (7) des primären Ballonkatheters (1) und mit dem Spülungslumen (15) des sekundären Ballonkatheters (2) verbunden ist, und
- eine Steuereinheit (25), die dafür konfiguriert ist, die Aufblas- und Ablasseinheit (20) und die Spülungsquelleneinheit (23) auf die folgende Weise zu steuern:
- Aufblasen des primären Ballons (4) durch die Aufblas- und Ablasseinheit (20),
- während des Platzierens des sekundären Ballonkatheters (2) in der Arbeitsposition oder danach Aufblasen des sekundären Ballons (13) durch die Aufblas- und Ablasseinheit (20),
- kontrollierte Freisetzung von Flüssigkeit durch das Spülungslumen (15) des sekundären Ballonkatheters (2) durch die Spülungsquelleneinheit (23) bis zum Erreichen eines Abbruchkriteriums,
- Ablassen des sekundären Ballons (13) durch die Aufblas- und Ablasseinheit (20),
- kontrollierte Freisetzung von Flüssigkeit durch eines von den Lumina (6 oder 7) des primären Ballonkatheters (1) durch die Spülungsquelleneinheit (23) bis zum Erreichen eines Abbruchkriteriums und
- Ablassen des primären Ballons (4) durch die Aufblas- und Ablasseinheit (20).

18. System nach Anspruch 17 mit der Doppelkatheteranordnung nach Anspruch 2 und 4,
wobei eine elektrische Messeinheit (24) zumindest mit der ersten und der dritten Elektrodenanordnung (9, 17) verbunden ist, die Mess-Signale liefern, die in der Steuereinheit (25) als eine Grundlage zum Feststellen des Abbruchkriteriums verwendet werden.

19. System nach Anspruch 18 mit der Doppelkatheteranordnung nach Anspruch 3 und 5,
wobei die elektrische Messeinheit (24) zumindest mit der zweiten und der vierten Elektrodenanordnung (10, 18) verbunden ist, die Mess-Signale liefern, die in der Steuereinheit (25) als eine Grundlage zum Feststellen des Abbruchkriteriums verwendet werden.

20. System nach einem der Ansprüche 17 bis 19,
wobei die Steuereinheit (25) dafür konfiguriert ist, die Spülungsquelleneinheit (23) derart zu steuern, dass zumindest eines von den mindestens zwei Lumina (6 oder 7) des primären Ballonkatheters (1) und dem Spülungslumen (15) des sekundären Ballonkatheters (2) unabhängig mit Kontrolle über Zeit, Druck, Durchfluss, Temperatur und/oder Art der Flüssigkeit gespült werden kann.

21. System nach einem der Ansprüche 17 bis 20,
wobei die Steuereinheit (25) dafür konfiguriert ist, die kontrollierte Freisetzung von Flüssigkeit durch eines von den Lumina (6 oder 7) des primären Ballonkatheters (1) durch die Spülungsquelleneinheit (23) rechtzeitig vor dem Ablassen des sekundären Ballons (13) durch die Aufblas- und Ablasseinheit (20) oder währenddessen zu beginnen.

## Revendications

1. Agencement de cathéter double et système de reperfusion d'une zone de tissu ischémique via un vaisseau coronaire, comprenant un cathéter à ballon primaire (1) doté d'un lumen (3) pour le gonflage et le dégonflage d'un ballon primaire (4) disposé dans une zone distale (5) du cathéter à ballon primaire (1) et un lumen (6) étendant à travers le cathéter à ballon primaire (1) et ouvert à une extrémité distale (8) du cathéter à ballon primaire (1), et un cathéter à ballon secondaire (2) doté d'un lumen (12) pour le gonflage et le dégonflage d'un ballon secondaire (13) disposé dans une zone distale (14) du cathéter à ballon secondaire (2), ledit cathéter à ballon secondaire (2) étant disposé à travers le lumen (6) cathéter à ballon primaire (1), de sorte que le cathéter à ballon secondaire (2) continue complètement à travers le lumen (6) du cathéter à ballon primaire (1) et s'étend distalement au-delà du cathéter à ballon primaire (1),
le cathéter à ballon primaire (1) comportant au moins un autre lumen séparé (7) s'étendant à travers le cathéter à ballon primaire (1) et ouvert à une extrémité distale (8) du cathéter à ballon primaire (1) dotée d'une bride de connexion (21) proximalement pour être détachable, d'une connexion étanche au fluide avec une unité de source de rinçage (23) et ouvert à l'extrémité distale (8) du cathéter à ballon primaire (1), et le cathéter à ballon secondaire (2) comportant un lumen d'irrigation (15) doté d'une bride de connexion proximalement pour être détachable (21), d'une connexion étanche au fluide avec une unité de source de rinçage (23) et d'une ouverture à une extrémité distale (16) du cathéter à ballon secondaire (2), ledit cathéter à ballon secondaire (2) créant, dans au moins une zone axiale le long de laquelle le ballon secondaire (13) est disposé, un moyen de renfort radial.

2. Agencement de cathéter double selon la revendication 1,
dans lequel un premier dispositif d'électrode (9) est fixé au moins à l'extrémité distale (8) du cathéter à ballon secondaire (1) et est conçu pour détecter une activité électrique d'un tissu entourant l'extrémité distale (8) du premier cathéter ballon (1).

3. Agencement de cathéter double selon la revendication 1 ou 2,
dans lequel un deuxième dispositif d'électrode (10) est fixé proximalement au ballon primaire (4) au moins dans des zones le long du cathéter à ballon primaire (1), et conçu pour détecter une activité électrique d'un tissu entourant le cathéter à ballon primaire (1).

4. Agencement de cathéter double selon l'une des revendications 1 à 3,
dans lequel un troisième dispositif d'électrode (17) est fixé à l'extrémité distale (8) du cathéter à ballon secondaire (2), et conçu pour détecter une activité électrique d'un tissu entourant l'extrémité distale (16) du cathéter à ballon secondaire (2).

5. Agencement de cathéter double selon l'une des revendications 1 to 4,
dans lequel un quatrième dispositif d'électrode (18) est proximalement (13) au moins dans des zones le long du cathéter à ballon secondaire (2), et conçu pour détecter une activité électrique d'un tissu entourant le second cathéter à ballon (2).

6. Agencement de cathéter double selon la revendication3 or 5,
dans lequel le deuxième positif d'électrode (10) apporte au moins deux électrodes (11) qui sont disposées à une distance l'une de l'autre le long d'une périphérie extérieure du cathéter à ballon primaire (1) et
le quatrième dispositif d'électrode (18) apporte au moins deux électrodes (19) qui sont disposées à une distance l'une de l'autre le long d'une périphérie extérieure du cathéter secondaire (2).

7. Agencement de cathéter double selon l'une des revendications 1 à 6,
dans lequel le cathéter à ballon secondaire (2) comporte une longueur de cathéter qui est supérieur à une longueur du cathéter à ballon primaire (1), de sorte que le cathéter ballon secondaire (2) dépasse le cathéter à ballon primaire (2) dans le sens distal à raison d'une longueur de jusqu'à 10 cm.

8. Agencement de cathéter double selon l'une des revendications 1 à 7,
dans lequel le cathéter à ballon secondaire (2) comporte un lumen pour un fil de guidage qui dépasse complètement axialement à travers le cathéter à ballon secondaire et s'ouvrant à l'extrémité distale (16) du cathéter à ballon secondaire (2).

9. Agencement de cathéter double selon la revendication 8,
dans lequel le lumen pour le fil de guidage s'étend au centre à travers le second cathéter à ballon et le lumen d'irrigation (15) et le lumen de gonflage et de dégonflage du ballon secondaire (13) sont disposés radialement autour du lumen pour le fil de guidage.

10. Agencement de cathéter double selon la revendication 8 ou 9,
dans lequel le lumen pour un fil de guidage a un diamètre constant allant de 300 à 1000 µm.

11. Agencement de cathéter double selon l'une des revendications 1 to 10,
dans lequel le cathéter à ballon secondaire (2) est conçu pour pouvoir glisser dans le lumen (6) du cathéter à ballon primaire (1), pour atteindre une position de travail dans laquelle le cathéter à ballon secondaire (2) continue entièrement à travers le lumen (6) du cathéter à ballon primaire (1) et s'étend distalement au-delà du cathéter à ballon primaire (1).

12. Agencement de cathéter double selon l'une des revendications 1 to 10,
dans lequel le cathéter à ballon secondaire (2) est disposé dans le lumen (6) du cathéter à ballon primaire (1) dans une position fixe dans laquelle le cathéter à ballon secondaire (2) continue complètement à travers le lumen (6) du cathéter à ballon primaire (1) et s'étend distalement au-delà du cathéter à ballon primaire (1).

13. Agencement de cathéter double selon l'une des revendications 1 to 12,
dans lequel le moyen de renfort radial (37) crée au moins une structure de forme annulaire qui est intégrée dans le cathéter à ballon secondaire (2) encerclant au moins le lumen d'irrigation (15).

14. Agencement de cathéter double selon la revendication 13,
dans lequel la structure de forme annulaire est monolithique ou a la forme d'une tresse.

15. Agencement de cathéter double selon la revendication 13 or 14,
dans lequel la structure de forme annulaire est composée d'au moins un des matériaux suivants : acier inoxydable, nitinol, PEEK, LCP, verre.

16. Agencement de cathéter double selon l'une des revendications 1 to 15,
dans lequel le cathéter à ballon primaire (1) comporte au moins un lumen d'aspiration ayant une ouverture à son extrémité distale.

17. Système de reperfusion d'une zone de tissu ischémique via un vaisseau coroner comprenant l'agencement de cathéter double selon l'une quelconque des revendications 1 à 16, comprenant en outre
- une unité de gonflage de dégonflage (20) connectée de manière étanche au fluide au lumen (3, 12) pour le gonflage et le dégonflage du ballon primaire et secondaire (1, 2),
- une unité de source de rinçage (23) connectée de manière étanche au fluide à l'autre lumen séparé (7) du cathéter à ballon primaire (1) et au lumen d'irrigation (15) du cathéter à ballon secondaire (2), et
- une unité de commande (25) configurée pour commander l'unité de gonflage et de dégonflage (20) et l'unité de source de rinçage (23) de la manière suivante
- gonflage du ballon primaire (4) par l'unité de gonflage et de dégonflage (20),
- pendant ou après le placement du cathéter à ballon secondaire (2) dans la position de travail, gonflage du ballon secondaire (13) par l'unité de gonflage et de dégonflage (20),
- déversement contrôlé de liquide à travers le lumen d'irrigation (15) du cathéter à ballon secondaire (2) par l'unité de source de rinçage (23) jusqu'à l'atteinte d'un critère d'interruption,
- gonflage du ballon secondaire (13) par l'unité de gonflage et de dégonflage (20),
- déversement contrôlé de liquide à travers l'un des lumens (6 ou 7) du cathéter à ballon primaire (1) par l'unité de source de rinçage (23) jusqu'à l'atteinte d'un critère d'interruption, et
- dégonflage du ballon primaire (4) par l'unité de gonflage et de dégonflage (20).

18. Système selon la revendication 17 avec l'agencement de cathéter double selon les revendications 2 et 4,
dans lequel une unité de mesure électrique (24) est connectée au moins au premier et troisième agencement d'électrodes (9, 17), en délivrant des signaux de mesure qui sont utilisés dans l'unité de commande (25) comme base pour déterminer le critère d'interruption.

19. Système selon la revendication 18 avec l'agencement de cathéter double selon les revendications 3 et 5,
dans lequel l'unité de mesure électrique (24) est connectée au moins au deuxième et quatrième agencement d'électrodes (10, 18) en délivrant des signaux de mesure qui sont utilisés dans l'unité de commande (25) comme base pour déterminer le critère d'interruption.

20. Système selon l'une quelconque des revendications 17 à 19,
dans lequel l'unité de commande (25) est configurée pour commander l'unité de source de rinçage (23) de manière à ce qu'au moins l'un des au moins deux lumens (6 ou 7) du cathéter à ballon primaire (1) et du lumen d'irrigation (15) du cathéter à ballon secondaire (2) puisse être rincé indépendamment avec un contrôle sur la durée, la pression, l'écoulement, la température et/ou le type de liquide.

21. Système selon l'une quelconque des revendications 17 à 20,
dans lequel l'unité de commande (25) est configurée pour lancer le déversement contrôlé de liquide à travers l'un des lumens (6 ou 7) du cathéter à ballon primaire (1) par la source de rinçage (23) temporellement avant ou pendant le dégonflage de ballon secondaire (13) par l'unité de gonflage et de dégonflage (20).
